(19)

Europäisches
Patentamt
European
Patent Office
Office européen
des brevets

(11) **EP 2 436 452 A1**

(12) **EUROPEAN PATENT APPLICATION**

(43) Date of publication:
**04.04.2012 Bulletin 2012/14**

(51) Int Cl.:
**B05B 7/00** (2006.01)

(21) Application number: **10181895.3**

(22) Date of filing: **29.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Unilever N.V.
3013 AL Rotterdam (NL)**

(72) Inventors:
• **Duggal, Charu
Whitefield
Bangalore 560 066 (IN)**

• **Naik, Vijay Mukund
Maharashtra
Mumbai 400 063 (IN)**
• **Raut, Janhavi Sanjay
Whitefield
Bangalore 560 066 (IN)**
• **Stoyanov, Simeon Dobrev
3133 AT Vlaardingen (NL)**

(74) Representative: **van Benthum, Wilhelmus A. J.
Unilever N.V.
Unilever Patent Group
Olivier van Noortlaan 120
3133 AT Vlaardingen (NL)**

(54) **Process for preparation of a foamed composition by hydrodynamic cavitation**

(57) The invention relates to a process for preparation of a foamed composition and a foamed composition prepared thereby. The invention more particularly relates to food compositions comprising gas bubbles that remain entrained for long periods of time in the compositions. According to the first aspect of the present invention there is provided a process to prepare a foamed composition comprising a liquid, gas bubbles dispersed therein and a foam stabilizer selected from di or multi block surface active polymers, surface active proteins, surface active colloidal particles and combinations there of, wherein the process comprises a step of foaming by hydrodynamic cavitation.

P

Figure - 1(a)

**EP 2 436 452 A1**

**Description**

**TECHNICAL FIELD**

The invention relates to a process for preparation of a foamed composition and a foamed composition prepared thereby. The invention more particularly relates to food compositions comprising gas bubbles that remain entrained for long periods of time in the compositions.

**BACKGROUND AND PRIOR ART**

There are many compositions that consumers use in which gas bubbles have been incorporated for providing many benefits which appeal to the consumer. Such products include cosmetics and foods. Foamed compositions find visual and sensory appeal in cosmetic compositions e.g. skin creams, shaving creams, hair products, liquid soaps etc. In the area of foods, gas bubbles have been incorporated for giving not just visual or sensory appeal to the products but for providing functional benefits. Many consumers prefer to eat large quantity of food but are unable to do so since they are weight conscious and want to reduce their intake of food that is high in carbohydrate, fat and other high calorie items. With such foods, foamed compositions have been prepared where the fat and carbohydrate contents have been reduced and replaced with gas bubbles. Such foamed food products give the consumer a feeling of satiety while ensuring minimization of calorie intake. The inclusion of gas e.g. air in the form of foams also provides for a unique mouth feel in many food products which are liked by many consumers. Further, the inclusion of foams in food products gives the product a different visual appeal, often quite attractive. Examples of such foamed food composition include, whipped cream, butter, and cheese which are consumed at room temperature; ice cream which is consumed at low temperature; and beverages like chocolate, coffee, milkshakes etc which may be consumed either hot or cold.

One of the problems with foamed compositions is that the foams tend to be unstable when the compositions containing them are stored over long periods of time e.g. over days and months. By the term unstable, is meant that the foam size tends to increase over time and all parts of a packaged composition, often, does not have the same amount of foam present in it. One example that many consumers have experienced is that while the top of the package on early use tends to be high in foam, the bottom of the package is flat and does not contain high amount of foam, as desired. Ways in which this stability problem have been tackled include incorporation of foam stabilizers in the foamed compositions. Commonly used foam stabilizers include emulsifiers which are surface active agents, proteins, and thickeners like gums e.g. alginates, gelatin, guar gums, pectins etc. These agents have had limited ability to stabilize the compositions and there are disadvantages in each one of them. While gums as thickeners have been the most common and successfully used foamed stabilizers, they have the inherent disadvantage of thickening the products thus making them unsuitable for use in products where high fluidity is desirable e.g. beverages. While, emulsifiers have less disadvantage in terms of reducing the fluidity of the product, they are not as good a foam stabilizer as gums. Thus, there has been a need in the art to develop materials that provide enhanced stabilization of foams in foamed compositions. The present inventors have been working on this for the last several years and a few prior patent applications from this group have been published in the last few years.

WO2008/046698 (Unilever) is a patent publication which discloses food composition comprising gas bubbles and processes for preparing them. This patent application discloses aerated food product in the form of a stable foam comprising 5-80 vol% gas bubbles, 15 - 90% water, and 0.001 to 10 wt% fibres assembled with surface active particles at the air water interface. A related patent application from the same research group in Unilever published as WO2008/046699 discloses similar food compositions comprising 10 to 95 wt% water, 0.5 to 20% protein, 1 to 70 vol% gas, 0.001 to 10 wt% fibre particles and 0.001 to 10% surface active particles. Frozen aerated food products have also been developed by Unilever and published as WO2008/046732 which discloses a product having an overrun of at least 30%, comprising 0.001 to 10 wt%, based on total weight of the product of surface active fibres, which have an aspect ratio of 10 to 1,000. Thus, while all of the above products have been significant improvements in producing foamed food compositions over the then prior art, the present group of researchers have been continuously working to improve them even further.

In developing the present invention, the inventors worked extensively on many process variations and finally hit upon a process viz. hydrodynamic cavitation in preparing such foamed compositions, which have not been extensively used in food processing. Surprisingly, this process provided certain benefits in both the size and shape of the gas bubbles with concomitant high stability of the foamed composition.

Hydrodynamic cavitation has been known and used in the chemical processing industry. It has also been known in producing mousse-like mixture of air and a foamable liquid (preferably a detergent) e.g. US5085371 (Shop Vac Corp, 1992). This publication discloses a nozzle assembly for producing a mousse-like mixture of air and a foamable liquid which includes a housing defining a passageway and a venturi located downstream of a mixing chamber also located in the passageway.

However, none of the published documents disclose that a unique combination of specific foam stabilizers which in combination with the selective process of generation of gas bubbles viz. hydrodynamic cavitation can be used for preparing a foamed composition where the foams have distinct bubble size/ shape and are also highly stable.

It is an object of the present invention to provide for a process to prepare a foamed composition where the foam principally comprises of gas bubbles having diameter in the range of 1 to 20 micrometer.

It is yet another object of the present invention to provide for a foamed composition having gas bubbles with diameter in the range of 1 to 20 micrometer where the foam is highly stable.

## SUMMARY OF THE INVENTION

According to the first aspect of the present invention there is provided a process to prepare a foamed composition comprising a liquid, gas bubbles dispersed therein and a foam stabilizer selected from di or multi block surface active polymers, surface active proteins, surface active colloidal particles and combinations there of, wherein the process comprises a step of foaming by hydrodynamic cavitation, wherein said hydrodynamic cavitation is created using a converging-diverging nozzle, and the process comprises the steps of (i) preparing a solution/dispersion comprising said liquid and said foam stabilizer and (ii) passing said solution/ dispersion through said converging-diverging nozzle using a pump, and wherein the Reynolds number at the throat of the converging diverging nozzle is at least 3500.

According to another aspect of the present invention there is provided a foamed composition prepared by the process of the invention.

According to yet another aspect of the present invention there is provided a foamed composition comprising (i) a liquid, (ii) gas bubbles dispersed therein having diameter in the range of 1 to 20 micrometer and (iii) a foam stabilizer selected from di or multi block surface active polymers, surface active proteins, surface active colloidal particles and combinations thereof.

## BRIEF DESCRIPTION OF THE FIGURES

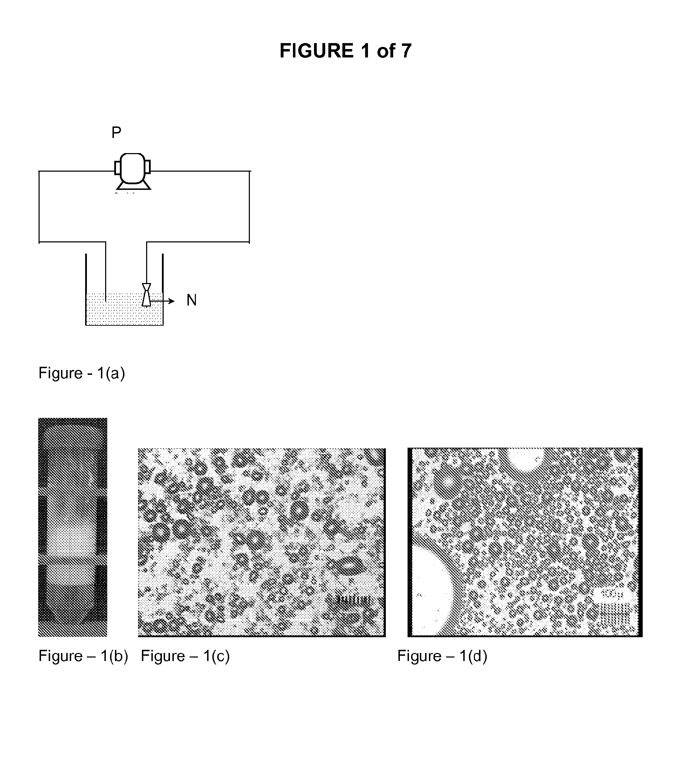

Figure - 1 (a) depicts a schematic of a device for preparing the foamed composition of the invention. Figure 1(b) shows the foamed composition while Figure 1(c) shows microscopy image of the fresh foam and Figure 1(d) the microscopy image after 30 days for Example 1

Figure 2(a) shows the microscopy picture of fresh foam and Figure 2(b) the microscopy picture of aged foam of Example 2 after 30 days.

Figure 3(a)-3(f): Foam height for the fresh and aged foams (stored for 15 days) prepared for Examples 3-8, respectively, using the different solutions/dispersions tabulated in Table 1.

Figures 4(a) and 4(b): Microscopy pictures of the bubbles obtained using hydrodynamic cavitation and conventional mixing of Examples 9 and 10, respectively.

Figure 5: Micrographs of the EC foam of Example 14, Figure: 5(a) EC Foam redispersed in DI water, (b) EC Foam redispersed in tomato sauce, (c) EC Foam redispersed in Mayonaise. Scale Bar =100 microns.

Figure 6:: Micrographs of the EC foam of Example 15; Figure 6(a) EC Foam redispersed in Body lotion, and (b) EC Foam redispersed in Body Cream. Scale Bar = 100 microns.

Figure 7(a), 7(b) 7(c) and 7(d): Microscopy pictures of the foam of Example 16 when sheared at RPM of 0, 6500, 13500 and 24000 min$^{-1}$, respectively

## DETAILED DESCRIPTION OF THE INVENTION

The invention provides for a process to prepare a foamed composition. The composition comprises a liquid, gas bubbles dispersed therein and a foam stabilizer. The liquid may be water, oil, solvents, gels, polymeric liquids, emulsions, suspensions or mixtures there of. The liquid is preferably water, solvent or oil, most preferably water. The gas which is present in the form of bubbles in the liquid is air, oxygen, nitrogen, nitrous oxide, helium, or carbon dioxide. The gas could also be any other gas which is relatively inert and have no adverse reaction when contacted with the outside surface of the human body or consumed internally. The more preferred gas present in the form of bubbles in the foamed composition is air or nitrogen, most preferably air. Thus, when air is the gas forming the foam, the composition is called aerated composition. Alternately, the gas may be introduced into the liquid in the dissolved form and could be replenished over cycles. The foam stabiliser is selected from di- or multi- block surface active polymers or surface active proteins, surface active colloidal particles and combinations thereof. By the term surface active is meant that the foam stabilizer when dissolved/ dispersed in water gives a gas/liquid surface tension of less then 50 mN/m, more preferably less than 40 mN/m. By multi block surface active polymer/proteins is meant block polymers or proteins that preferentially reside at the interface due to their surface energy charateristics or due to presence of both hydrophobic and hydrophilic moeties. An example of proteins that can provide foam stability for more than a week of storage is bovine albumin.

According to a preferred aspect of the invention the foam stabiliser is a surface-active particle which is selected from the group consisting of modified celluloses, modified starches, proteins, modified inorganic particles or mixtures thereof.

By surface active colloidal particles is meant particles that can stabilize the interface between gas and the liquid. These can be homogeneous particles with appropriate surface energies as well as bimodal (Janus) particles exhibiting both hydrophilic and hydrophobic behaviours. Examples of particles that provide foam stability for more than a week of storage include ethyl cellulose, microcrystalline cellulose, or mixtures thereof.

The foam stabilizer is preferably a surface-active particle with a contact angle between 60° and 120°, more preferably between 70° and 110°, further more preferably between 80° and 100°.

The foam stabilizer is preferably a surface-active particle with a volume weighted mean diameter in the range of 0.01 to 10 $\mu$m more preferably in the range of 0.01 to 1 $\mu$m.

From the above classes of foam stabilizers, the most preferred ones are albumins, microcrystalline cellulose and ethyl cellulose particles. When ethyl cellulose particles are used as the foam stabilizer, it is further preferred that they are used along with microcrystalline cellulose.

The essential step of producing the foam in the composition is hydrodynamic cavitation. Cavitation is the formation and subsequent implosion of bubbles or cavities in a liquid. When the local pressure in a liquid becomes lower than the vapour pressure of the liquid (at that temperature) or when the local pressure turns negative (signifying tension rather than compression), small bubbles or cavities form. These cavities may be entirely newly formed when the negative pressures are large enough to overcome the attractive van der Waals forces between molecules. Theoretically, one can show that, for water, this would occur if the negative pressure is about 4000 N/m$^2$. Alternatively (and realistically), small pre-existing bubbles may act as nuclei, leading to bubble expansion. On reaching high pressure regions, these bubbles can collapse catastrophically, leading to generation of high pressures, temperatures and velocities. Cavitation is usually of two types - hydrodynamic cavitation, produced by pressure variations in a flowing liquid due to the geometry of the system, and the more familiar acoustic cavitation, produced by sound waves in a liquid. Hydrodynamic cavitation in flowing systems can be created through the use of convergent-divergent nozzles geometries where the flowrate is adjusted such that the velocity head increases significantly thereby reducing the pressure head below the vapour pressure of the fluid.

The hydrodynamic cavitation, in the present invention is preferably created using a converging-diverging nozzle. Thus, a suitable way of preparing the foamed composition of the invention comprises taking the desired liquid in a vessel. The foam stabilizer is then dissolved/ dispersed in the liquid through appropriate means. If the foam stabilizer is insoluble in the liquid, it may be dispersed in the liquid through use of a second liquid in which the foam stabilizer is soluble. The second liquid may be an organic solvent. Suitable second liquids are ethanol or acetone. Once the foam stabliser, the desired liquid and the second liquid are well mixed, the second liquid may be suitably removed from the mixture by any known method. If the second liquid is a volatile organic solvent, it may be removed by evaporation under vacuum. The mixture of the desired liquid and the foam stabilizer is then taken in a vessel in which the converging-diverging nozzle is immersed. The mixture is then recirculated through the converging-diverging nozzle through a suitable pump. Suitable pumps are positive displacement pumps like peristaltic or gear pumps which can build suitable head pressure to provide the required flowrates. Centrifugal pumps can also be used for the process. The mixture may be circulated for a sufficient period of time to simulate a single pass through the pump or may be long enough to encompass multiple passes. Alternatively dissolved gases and/or small pre-existing bubbles can act as nuclei, leading to bubble expansion. The bubbles which thereby constitute both the vapour and the gases once formed get stabilized by the foam stabilizer present in the liquid. Figure - 2 (a) depicts a schematic of a device for preparing the foamed composition of the invention.

It is preferred that the gas bubbles have diameter less than 20 micrometer, preferably in the range of 1-20 micrometer.

Thus, a preferred process comprises the steps of (i) preparing a solution/dispersion comprising the liquid and the foam stabilizer and (ii) passing said solution/ dispersion through said converging-diverging nozzle using a pump.

The Reynolds number at the throat of the converging diverging nozzle is at least 3500, preferably at least 5000, more preferably at least 10000. It is particularly preferred that the Reynolds number at the throat of the converging diverging nozzle is greater than 12500, or even more preferably greater than 15000. The term throat as used herein means the region between the converging and the diverging sections where the cross-sectional area for the flow is the smallest.

**The Reynolds number at the throat is defined as**

$Re = D\rho U/\mu$

Where D is hydraulic diameter at the throat, defined as 4A/P where A is the cross-sectional area at the throat and P is the wetted perimeter.

U is the liquid velocity at the throat

$\mu$ is the viscosity of the liquid

$\rho$ is the density of the liquid

The solution/ dispersion is preferably passed through the converging-diverging nozzle at a velocity greater than 10 m/s, more preferably greater than 15 m/s. The driving pressure is determined by the wall friction and this is preferably low enough to permit a sufficiently low pressure at the throat region for cavitation to occur. The low pressure requirement at the throat can be increased through increase in temperature or use of volatile solvents, dissolved gases or seeding agents. The wall friction can be reduced through use of appropriate material of construction, wall geometries as well as drag reducing agents. The inlet/driving pressure (gauge) at which the solution/ dispersion is passed through the converging-diverging nozzle is preferably in the range of $10^4$ to $10^7$ N/m$^2$, more preferably in the range of $10^4$ to $10^6$ N/m$^2$.

It is preferred that external gas phase introduced in the liquid is less than 10% by volume of the liquid phase. The term "external gas phase" as used herein excludes the bubbles formed due to cavitation. It is further preferred that external gas phase introduced in the composition is less that 5% by volume of the liquid phase. It is particularly preferred that no external gas phase is introduced during the process. Preferably, the converging section does not have any inlet for external gas such that no external gas is sucked in by the Venturi effect during step (ii).

The process of the invention may optionally comprise additional steps subsequent to the essential steps of the process of the present invention. Optional steps may be cooling, freezing, gellation or structuring.

According to another aspect of the present invention there is provided a foamed composition prepared by a process of the invention. The composition prepared by the process of the present invention preferably has the gas bubbles in a substantially spherical or elongate shape. Foamed composition prepared by the process of the present invention preferably has the diameter of the gas bubbles less than 20 micrometer, preferably in the range of 1 to 20 micrometer. In the present invention, the diameter of an ellipsoidal (elongated) bubble is the minor diameter.

The foamed composition of the invention may comprise the foam stabilizer, the liquid and the gas bubbles over a large range of concentration depending upon the use of the composition. In general, it is preferred that the foam stabilizer is in 0.01 to 20 %, more preferably 1 to 5% by weight of the composition. The liquid is preferably present in 10 to 95%, more preferably 10 to 50% by volume of the composition. The gas bubbles are preferably present in 5 to 90 %, more preferably 50 to 80% by volume of the composition.

The foamed composition has applications over a wide range of consumer products, most preferred being food products or cosmetic products e.g. for application on skin, hair or other external surfaces of the body.

The present invention is especially suitable for preparing food product. Food product that may be prepared include whipped cream, cheese, butter or fat, ice-cream, soups, sauces, yoghurts, custards, spreads, chocolates or beverage compositions comprising coffee, tea milkshakes or buttermilk.

When the product is a frozen product, it is prepared by the process of the invention to make a foamed composition that comprises liquid, gas bubbles dispersed therein and the foam stabilizer. The foamed composition may then be cooled or frozen to the desired temperature to prepare the frozen product which may be a gel, a soft or hard solid. In the final frozen product, it is possible that the liquid originally subjected to the process of the invention is in the solid state e.g. in the form of ice crystals.

According to yet another aspect of the present invention there is provided a foamed composition comprising (i) a liquid, (ii) gas bubbles dispersed therein having diameter in the range of 1 to 20 micrometer and (iii) a foam stabilizer selected from, multi block surface active polymers, surface active proteins, surface active colloidal particles and combinations thereof. In this aspect of the present invention, the foam stabilizer is preferably selected from albumins, ethyl cellulose or microcrystalline cellulose.

The composition of the invention has a %overrun which is preferably is in the range of 2 to 200%, more preferably in the range of 10 to 100%. The term "overrun" refers to the ratio of the gas volume in the foam and the volume of the liquid involved in its formation expressed as a percentage value. Thus,

$$\%Overrun = [(V_{total} - V_{liquid})/V_{liquid}] \times 100\%$$

where $V_{liquid}$ = initial volume of the liquid and

$V_{total}$ = final volume of the liquid-gas mixture after aeration. The %overrun thus refers to the extent of gas incorporated through the process.

The invention will now be illustrated with the help of the following non-limiting examples.

**EXAMPLES**

Examples 1-2: Process for creation of foamed compositions as per the invention

Example -1: Ethyl Cellulose as foam stabiliser and using step of Hydrodynamic Cavitation

2% dispersion of Ethyl Cellulose (Product No. 38330, LR grade from S.D. Fine Chem Limited) was prepared by dissolving 1 wt% of Ethyl Cellulose (EC) powder into acetone at 30°C. Subsequently, the equal volume of water was quickly added into the EC solution under strong stirring to precipitate the EC into colloidal particle form. The acetone and half the water was then removed by using a rotary evaporator to obtain a 2% dispersion.

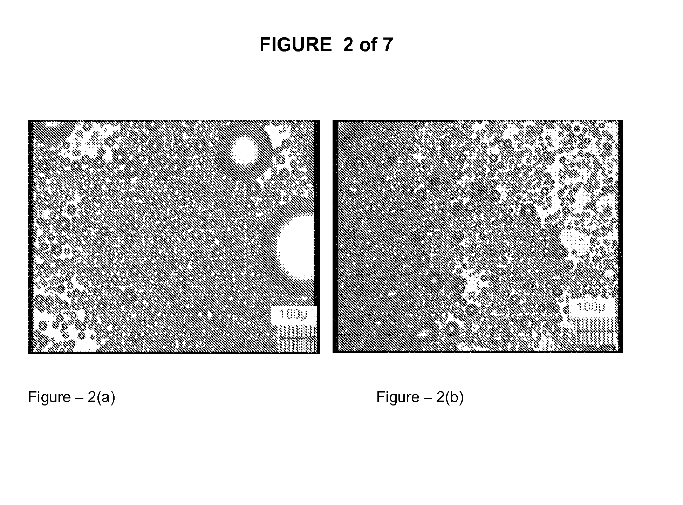

100 ml of the solution was circulated through a cavitation nozzle using the process scheme as shown in Figure 1(a) to subject the solution to hydrodynamic cavitation. The nozzle outlet was immersed entirely in the fluid mass to avoid air entrainment through surface turbulence. The flow through the nozzle was at 1.5 liter/minute and a head of 2.0 atmosphere gauge. The diameter at the throat was 1 mm. The Reynolds number at the throat was 15900. The nozzle geometry was convergent-divergent as shown in Figure 2(a). Under the conditions described above, cavitating flow was seen to take place in the nozzle. The solution was circulated for 10 minutes. In this time about 50% overrun was observed. The foam was observed to be stable up to 30 days. Figure 1(b) shows the foamed composition while Figure 1(c) shows microscopy image of the fresh foam and Figure 1(d) the microscopy image after 30 days. The ethyl cellulose foam was stored at ambient conditions. The height of the foam in Figure 1(b) did not decrease even after storage for 30 days. Comparison of the Figure 1(c) and 1(d) indicates that the bubble size did not change substantially even after storage for 30 days. Thus foamed compositions can be prepared by the process of the invention using ethyl cellulose as the foam stabiliser.

Example 2: Albumin as foam stabiliser and using step of Hydrodynamic Cavitation

100 ml of 5% solution of Bovine Albumin Serum {BSA 5 Albumin, Fraktion 5 (Lyophilisat)} was prepared by dissolving 5 grams of BSA in water at 50 °C by stirring for 50 minutes and subsequently cooling to room temperature. The solution was circulated through the converging - diverging nozzle using the process scheme as shown in Figure 1 (a) for 10 minutes. The diameter at the throat was 1 mm. The Reynolds number was 15900. The cavitation resulted in a overrun of 20%. The foam was stable up to 30 days. Figure 2(a) shows the microscopy picture of fresh foam and Figure 2(b) the microscopy picture of aged foam after 30 days. The foam was stored at -5 °C. Thus, foamed compositions can be prepared by the process of the invention using albumin as the foam stabiliser.

Examples 3 to 8: Stability of foam created using Hydrodynamic Cavitation for different stabilizers.

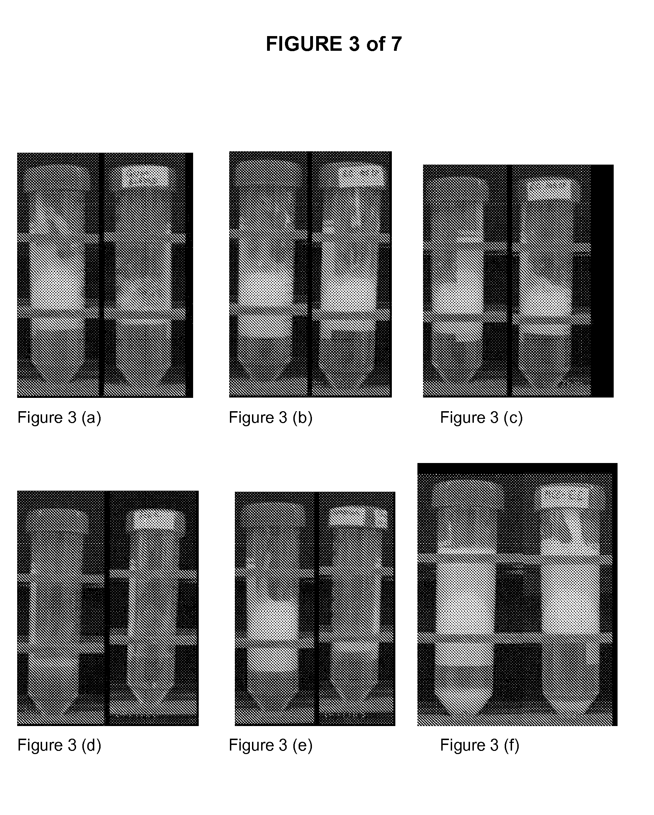

Solutions/dispersions of various foam stabilizers used in food products as listed in Table - 1 were used for creating foams using hydrodynamic cavitation using the process scheme as shown in Figure 1 (a). The solutions/ dispersions were circulated for 7 minutes. Reynolds number was between 10000 and 12500. The foams were collected in calibrated tubes and the foam height observed over a period of time. Figure 3(a) to 3(f) shows the foam height for the fresh and aged foams (stored for 15 days) prepared using the different solutions/dispersions.

Table - 1

| Example | Foam Stabiliser | Within the invention? | Concentration , Wt% | Figure depicting Foam |
|---|---|---|---|---|
| 3 | Gum blend* | No | 0.2% | 3(a) |
| 4 | Ethyl Cellulose LR grade [Product No. 38330] from SD.Fine Chem Limited | Yes | 2% | 3(b) |
| 5 | 2% dispersion of Ethyl Cellulose [200662] from Aldrich | Yes | 2% | 3(c) |

(continued)

| Example | Foam Stabiliser | Within the invention? | Concentration , Wt% | Figure depicting Foam |
|---------|-----------------|----------------------|---------------------|----------------------|
| 6 | Hydroxyethyl cellulose [09368 from Fluka, Biochemica | No | 0.5% | 3(d) |
| 7 | Methocel Food Grade Hydroxy Propyl Cellulose [E464 from the Dow Chemical Co. | No | 0.5% | 3 (e) |
| 8 | Ethyl Cellulose (EC) [200662-500g] from Aldrich + Micro Crystalline Cellulose (MCC) | Yes | 2% EC + 1% MCC | 3(f) |

* Gum blend here is a blend of glycerol mono stearate (60%), Locust bean gum (24%), Guar Gum (12%), Carrageenan (4%).

In Table-1, the foam stabilizers of Examples 3, 6, and 7 are not in the class of selected foam stabilizers of the invention viz. di or multi- block surface active polymers or surface active proteins, or surface active colloidal particles.

The Figures 3(a) to 3(f) indicate that use of foam stabilisers as per the invention provide for stable foams (see Figure 3 (b), 3 (c) and 3(f) for examples 4, 5 and 8) while examples outside the invention i.e. Examples 3, 6, and 7 (Figures 3(a), 3(d) and 3(e) give poor foam stability.

Examples 9 - 10: Bubble size obtained in foamed compositions with step of hydrodynamic cavitation as compared to conventional mixing step

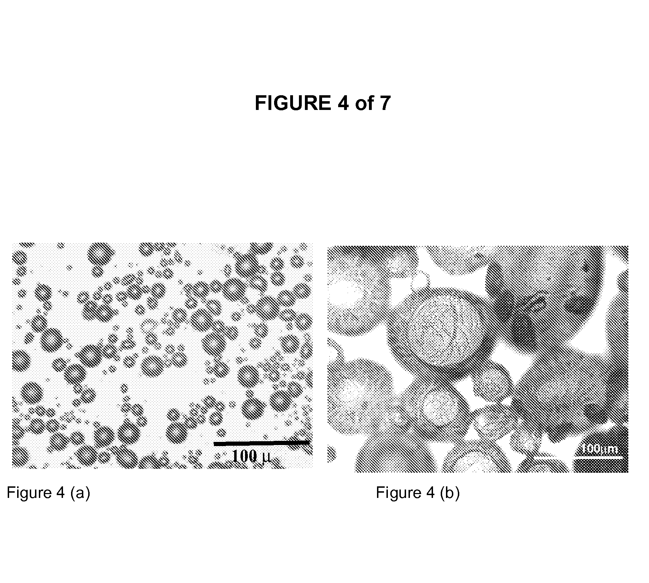

Various experiments were conducted to determine the characteristics of foamed compositions using the step of hydrodynamic cavitation in comparison to conventional mixing step. These experiments were conducted as follows: In the process comprising the step of hydrodynamic cavitation, dispersions of the foam stabiliser were circulated through a cavitation nozzle using the process scheme as shown in Figure 1(a). In the conventional process the same dispersion was whipped using a Kenwood Kitchen mixer at full power for 2 minutes. The various experiments are summarised in Table - 2 and the results are depicted in Figures 4(a) and 4(b) in terms of microscopy pictures of the bubbles obtained using the two techniques.

Table - 2

| Example | Process step | Foam Stabiliser | Concentration of foam stabiliser | Result in Figure |
|---------|-------------|-----------------|----------------------------------|------------------|
| 9 | Hydrodynamic cavitation | Ethyl cellulose | 2% | 4(a) |
| 10 | Conventional process | Ethyl cellulose | 2% | 4(b) |

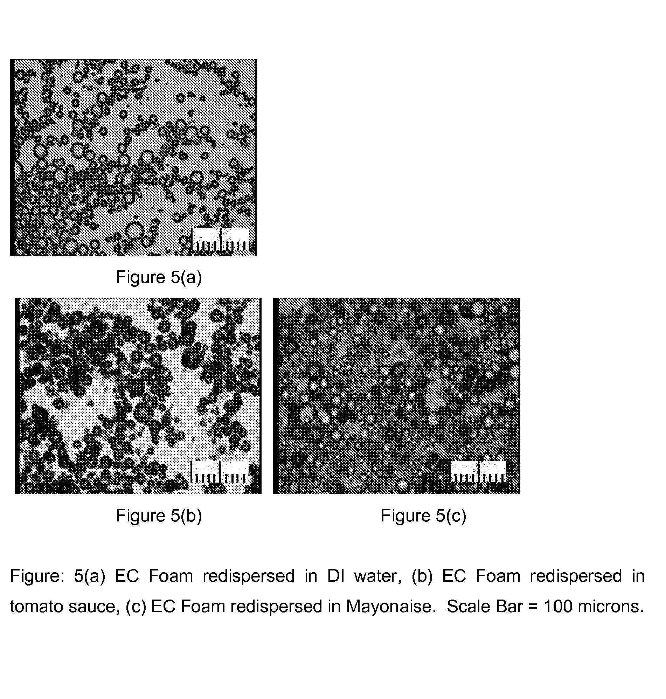

The Figures 4(a) and 4(b) indicate that process as per the invention (Figures 5(a)) provides for foamed composition with much smaller bubble sizes as compared to conventional process.

Examples 11-13: Effect of Reynolds number at the throat

The Experiments were carried out in a manner similar to that of Example 1 except that the flowrate was varied. The results are tabulated below (Example 1 is reproduced for convenience).

| Example No | Flowrate (L/min) | U (m/s) | Reynolds number at the throat (dimensionless) | % Overrun (after 10 minutes) |
|-----------|-----------------|---------|----------------------------------------------|------------------------------|
| 11 | 0.25 | 5.3 | 2650 | 0 |
| 12 | 0.5 | 10.6 | 5300 | 5 |
| 13 | 1.2 | 21.2 | 10600 | 30 |

(continued)

| Example No | Flowrate (L/min) | U (m/s) | Reynolds number at the throat (dimensionless) | % Overrun (after 10 minutes) |
|---|---|---|---|---|
| 1 | 1.5 | 31.8 | 15900 | 50 |

From the results, it is clear that when Reynolds number at the throat is less than 9000, foam is not formed. The amount of foam (as indicated %overrun) increases with the increase in the Reynolds number.

Example 14:

Introduction of Preformed EC Foam into Food Products:

EC Foam was prepared as per the process described in Example 1. The stored foam was redispersed into foods products like tomato sauce (Maggie® - Nestle) and Mayonaise (Classic Creamy®- Unilever) by stirring in the foam using a spatula. The ratio of foam to product was 50% by volume. The aerated product was observed under the microscope. Figures 5(a), 5(b) and 5(c) show micrographs of the EC foam redispersed in deionised water, foam redispersed in tomato sauce and foam redispersed in mayonnaise, respectively. As seen in the figures, the bubble size is conserved in the high viscosity complex products as well.

Example 15:

Introduction of Preformed EC Foam into Personal Care Products:

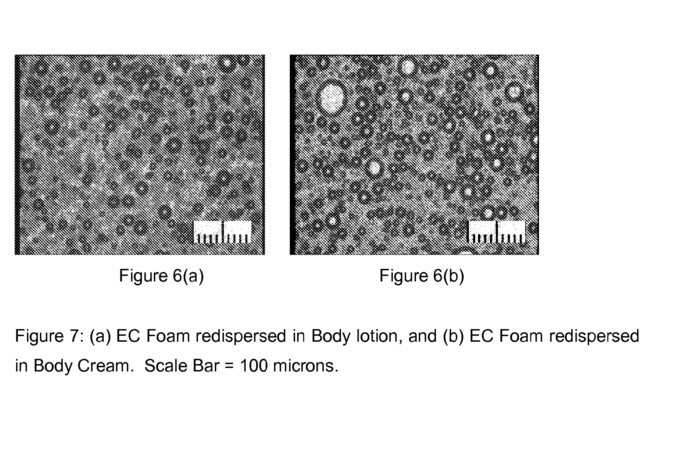

EC Foam was prepared as per the process described in Example 1. The stored foam was redispersed into Personal care products like body lotion and body cream (GIVE BRANDS) by stirring in the foam using a spatula. The ratio of foam to product was 50% by volume. The aerated product was observed under the microscope. Figures 6(a) and 6(b) show micrographs of the EC foam redispersed in body lotion and in body cream, respectively. As seen in the figures, the bubble size is conserved even after dispersion into these complex viscous fluids.

Example 16:

Effect of high shear on the Preformed EC Foam:

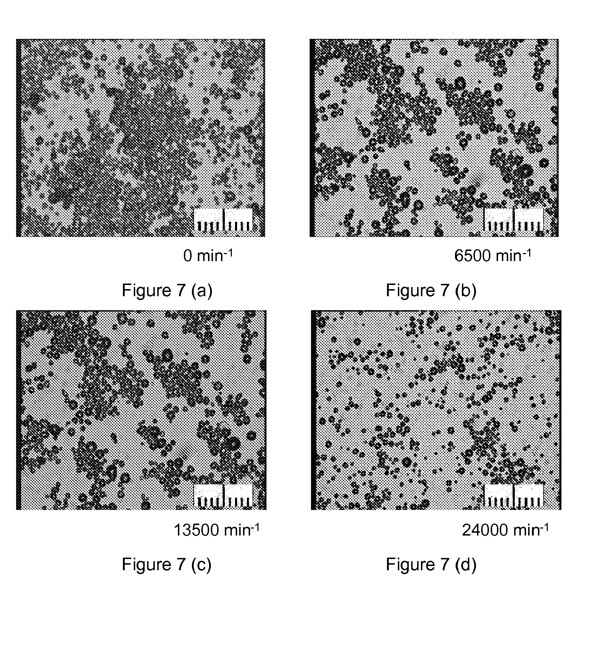

EC Foam was prepared as per the process described in Example 1. The foamed dispersion was subjected to high shear using a High speed Homogenizer (Ultra Turraz T25). The RPM was varied from 0 to 24000 min$^{-1}$. The foamed dispersion was sheared for 5 minutes making sure that no further air was entrained during the process. The EC foam was observed under the microscope. Figures 7(a), 7(b), 7(c) and 7(d) show the microscopy pictures of the foam when sheared at RPM of 0, 6500, 13500 and 24000 min$^{-1}$, respectively. As seen from the figures the bubble size remains unchanged even after subjecting to high shear.

**Claims**

1.  A process to prepare a foamed composition comprising a liquid, gas bubbles dispersed therein and a foam stabilizer selected from di or multi- block surface active polymers or surface active proteins, surface active colloidal particles and combinations thereof, wherein the process comprises a step of foaming by hydrodynamic cavitation, wherein said hydrodynamic cavitation is created using a converging-diverging nozzle, and the process comprises the steps of

    (i) preparing a solution/dispersion comprising said liquid and said foam stabilizer and (ii) passing said solution/ dispersion through said converging-diverging nozzle using a pump, and wherein the Reynolds number at the throat of the converging diverging nozzle is at least 3500.

2.  A process as claimed in claim 1 wherein said converging section does not have any inlet for external gas such that no external gas is sucked in by the Venturi effect during step (ii).

3.  A process as claimed in claim 1 or claim 2 wherein said solution/ dispersion is passed through said converging-diverging nozzle at a maximum velocity greater than 10 m/s.

4. A process as claimed in any one of the preceding claims wherein said solution/dispersion is passed though said converging-diverging nozzle at a gauge pressure in the range of $10^4$ to $10^7$ N/m$^2$.

5. A process as claimed in any one of the preceding claims wherein said liquid is water, solvent, or oil.

6. A process as claimed in any one of the preceding claims wherein said gas bubbles comprise air, oxygen, nitrogen, nitrous oxide, helium, or carbon dioxide.

7. A process as claimed in any one of the proceding claims wherein said foam stabilizer is selected from albumins, microcrystalline cellulose or ethyl cellulose particles.

8. A process as claimed in any one of the preceding claims wherein said gas bubbles have diameter in the range of 1 to 20 micrometer.

9. A process as claimed in any one of the preceding claims wherein said foam stabilizer is present in 0.01 to 20 % by weight of the composition.

10. A foamed composition prepared by a process as claimed in any one of the preceding claims.

11. A foamed composition as claimed in claim 10 for application in foods or cosmetics.

12. A composition as claimed in claim 11 wherein said food is whipped cream, cheese, butter or fat, ice-cream, soups, sauces, yoghurts, custards, spreads, chocolates or beverage compositions comprising coffee, tea, milkshakes or buttermilk.

13. A foamed composition as claimed in any one of the preceding claims 10-12 comprising (i) a liquid, (ii) gas bubbles dispersed therein having diameter in the range of 1 to 20 micrometer and (iii) a foam stabilizer selected from di or multi block surface active polymers, surface active proteins, surface active colloidal particles and combinations there.

14. A foamed composition as claimed in claim 13 wherein said foam stabilizer is selected from albumins, ethyl cellulose or microcrystalline cellulose.

# FIGURE 1 of 7

P

N

Figure - 1(a)

Figure – 1(b)  Figure – 1(c)

Figure – 1(d)

# FIGURE 2 of 7

Figure – 2(a)                                                    Figure – 2(b)

# FIGURE 3 of 7

Figure 3 (a)

Figure 3 (b)

Figure 3 (c)

Figure 3 (d)

Figure 3 (e)

Figure 3 (f)

**FIGURE 4 of 7**

Figure 4 (a)                    Figure 4 (b)

Figure 5(a)

Figure 5(b)

Figure 5(c)

Figure: 5(a) EC Foam redispersed in DI water, (b) EC Foam redispersed in tomato sauce, (c) EC Foam redispersed in Mayonaise. Scale Bar = 100 microns.

Figure 6(a)          Figure 6(b)

Figure 7: (a) EC Foam redispersed in Body lotion, and (b) EC Foam redispersed in Body Cream.  Scale Bar = 100 microns.

0 min⁻¹

Figure 7 (a)

6500 min⁻¹

Figure 7 (b)

13500 min⁻¹

Figure 7 (c)

24000 min⁻¹

Figure 7 (d)

Europäisches
Patentamt

European
Patent Office

Office européen
des brevets

**EUROPEAN SEARCH REPORT**

Application Number

EP 10 18 1895

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 2 750 347 A1 (KLEIN, CHRISTOPHE PIERRE LUCIEN [FR]) 2 January 1998 (1998-01-02) * page 3, line 23 - page 10, line 34; figure 1 * ----- | 1,3-14 | INV. B05B7/00 |
| A | WO 02/076624 A1 (DUSHKIN, ANDREY L. [RU]; KARPYSHEV, ALEXANDER V. [RU]) 3 October 2002 (2002-10-03) * page 7, line 2 - page 13, line 26; figures 1-4 * ----- | 1 | |
| A | GB 760 765 A (GENERAL FOODS CORP [US]) 7 November 1956 (1956-11-07) * page 3, left-hand column, line 64 - page 4, left-hand column, line 17 * ----- | 7 | |

| | | | TECHNICAL FIELDS SEARCHED (IPC) |
|---|---|---|---|
| | | | B05B A23L |

The present search report has been drawn up for all claims

| Place of search | Date of completion of the search | Examiner |
|---|---|---|
| Munich | 28 February 2011 | Innecken, Axel |

EPO FORM 1503 03.82 (P04C01)

**ANNEX TO THE EUROPEAN SEARCH REPORT
ON EUROPEAN PATENT APPLICATION NO.**

EP 10 18 1895

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2011

| Patent document cited in search report | | Publication date | Patent family member(s) | | Publication date |
|---|---|---|---|---|---|
| FR 2750347 | A1 | 02-01-1998 | NONE | | |
| WO 02076624 | A1 | 03-10-2002 | AP | 1570 A | 28-02-2006 |
| | | | AT | 298634 T | 15-07-2005 |
| | | | AU | 2002251620 B2 | 03-11-2005 |
| | | | BR | 0208293 A | 13-04-2004 |
| | | | CA | 2441405 A1 | 03-10-2002 |
| | | | CN | 1498137 A | 19-05-2004 |
| | | | DE | 60204857 D1 | 04-08-2005 |
| | | | DE | 60204857 T2 | 18-05-2006 |
| | | | EP | 1370367 A1 | 17-12-2003 |
| | | | ES | 2244766 T3 | 16-12-2005 |
| | | | HK | 1066186 A1 | 25-08-2006 |
| | | | JP | 4065410 B2 | 26-03-2008 |
| | | | JP | 2004532721 T | 28-10-2004 |
| | | | MX | PA03008600 A | 07-03-2005 |
| | | | NZ | 528574 A | 24-03-2005 |
| | | | OA | 12593 A | 08-06-2006 |
| | | | PT | 1370367 E | 30-11-2005 |
| | | | RU | 2184619 C1 | 10-07-2002 |
| | | | US | 2004124269 A1 | 01-07-2004 |
| | | | ZA | 200307341 A | 14-07-2004 |
| GB 760765 | A | 07-11-1956 | NONE | | |

EPO FORM P0459

For more details about this annex : see Official Journal of the European Patent Office, No. 12/82

**EP 2 436 452 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008046698 A
- WO 2008046699 A
- WO 2008046732 A
- US 5085371 A